# EUROPEAN PATENT APPLICATION

(11) **EP 4 417 205 A1**
(43) Date of publication of application: **21.08.2024**
(21) Application number: 22881411.7
(22) Date of filing: 14.10.2022
(51) Int. Cl.: A61K 31/496, A61K 31/635, A61K 31/7068, A61K 31/706, A61K 45/06, A61P 35/02

(54) **COMPOSITION FOR COMBINATION THERAPY, COMPRISING 2,3,5-SUBSTITUTED THIOPHENE COMPOUND**

(30) Priority: 14.10.2021 KR 20210136944
(71) Applicant: Pharos Ibio Co., Ltd, Anyang-si, Gyeonggi-do 14059 (KR)
(72) Inventor: YOON, Jeong Hyeok, Yongin-si Gyeonggi-do 16902 (KR); NAM, Ky Youb, Goyang-si Gyeonggi-do 10419 (KR)
(74) Representative: Cabinet Nony
(86) International application number: PCT/KR2022/015621
(87) International publication number: WO 2023/063784

(57) **Abstract**

The present invention relates to a composition for co-administration containing a 2,3,5-substituted thiophene compound. The composition has excellent inhibitory activity against cancer cells related to leukemia compared to treatment with the 2,3,5-substituted thiophene compound alone or an anticancer drug alone, and thus may be useful for the prevention, amelioration or treatment of leukemia.

## Description

### Technical Field

The present invention relates to a composition for co-administration containing a 2,3,5-substituted thiophene compound.

### Background Art

With the development of modern medicine, many diseases have been treated and prevented, but cancer is still one of diseases difficult to treat. Currently, cancer is the first leading cause of death and continues to increase.

As a method for treating cancer, chemotherapy, surgical therapy and/or radiotherapy, etc. are used. Among them, chemotherapy is a method using anticancer drugs and is most often used in the treatment of cancer. Today, about 60 kinds of various anticancer drugs are being used in clinical practice, and new anticancer drugs are continuously being developed as knowledge about cancer incidence and characteristics of cancer cells is sufficiently known. However, most of anticancer drugs that are currently used in clinical practice often cause adverse effects such as nausea, vomiting, ulcers of the oral cavity and small intestine, diarrhea, hair loss, and/or myelosuppression that results in the decreased production of blood active ingredients. For example, it is known that mitomycin-C has side effects such as renal failure, and adriamycin has adverse effects such as myelosuppression. In particular, cisplatin, which is the most useful drug among anticancer drugs developed so far, is widely used in the treatment of testicular cancer, ovarian cancer, lung cancer, head and neck cancer, bladder cancer, gastric cancer, and cervical cancer, but has the big problem of showing adverse effects such as hematopoietic toxicities such as anemia, gastrointestinal toxicities such as vomiting and nausea, kidney toxicities such as kidney tubule damage, hearing loss, abnormalities in body electrolytes, shock, and peripheral nerve abnormalities.

New high-priced targeted anticancer drugs having excellent safety, which have recently been developed, exhibit efficacy in the treatment of many cancer patients, but exhibit efficacy only in a small group of patients by companion diagnosis. Therefore, there is an urgent need for precision medicine enabling personalized treatment in various cancer types.

Leukemia refers to all diseases in which leukocytes proliferate neoplastically. Types of leukemia are classified into myeloid leukemia and lymphocytic leukemia based on the leukocytes from which the leukemia originates, and are also classified into acute leukemia and chronic leukemia based on the rate of progress. The clinical patterns of leukemia vary depending on the type of disease and the characteristics of the invaded cells. Lymphocytic leukemia is caused by mutation of lymphocytic blood cells, and myeloid leukemia is caused by mutation of myeloid blood cells. In addition, chronic myeloid leukemia is caused by mutation of cells in the mature stage, and acute myeloid leukemia is caused by a disorder of myeloid progenitor cells that begin to differentiate during the relatively early stage of hematopoiesis.

Accordingly, the present inventors have conducted studies to discover an effective formulation for the treatment of leukemia, thereby completing the present invention.

### DISCLOSURE

### Technical Problem

An object of the present invention is to provide a pharmaceutical composition for preventing or treating leukemia, containing a compound represented by the following Formula 1 or a pharmaceutically acceptable salt thereof and an anticancer drug:

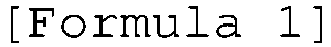

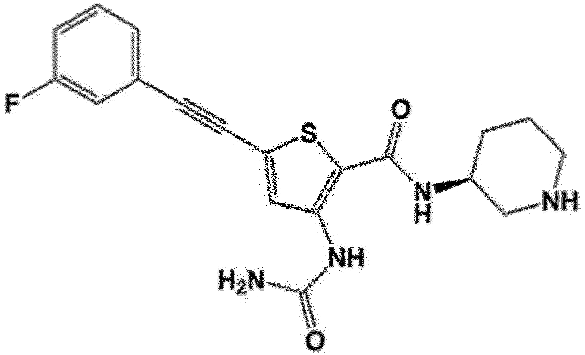

Another object of the present invention is to provide a method for treating leukemia, comprising a step of administering the pharmaceutical composition to a leukemia patient.

Still another object of the present invention is to provide the use of a compound represented by the following Formula 1 for preventing or treating leukemia:

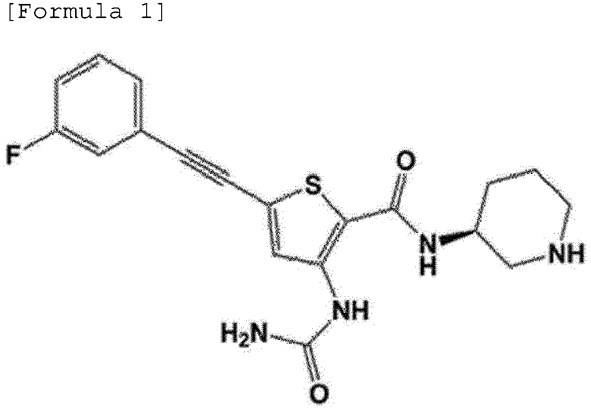

Yet another object of the present invention is to provide the use of a compound represented by the following Formula 1 for preparing a pharmaceutical composition for preventing or treating leukemia:

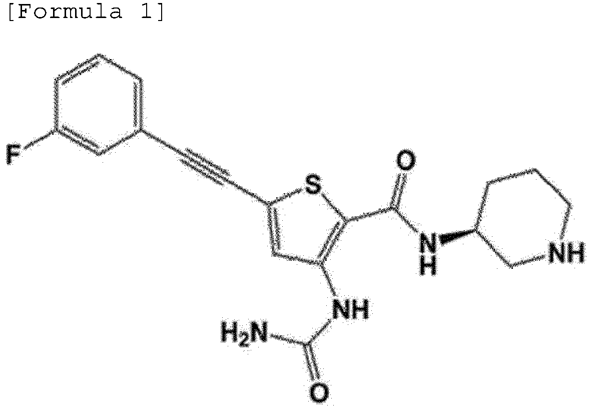

### Technical Solution

To achieve the above objects, one aspect of the present invention provides a pharmaceutical composition for preventing or treating leukemia, containing a compound represented by the following Formula 1 or a pharmaceutically acceptable salt thereof and an anticancer drug:

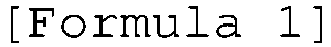

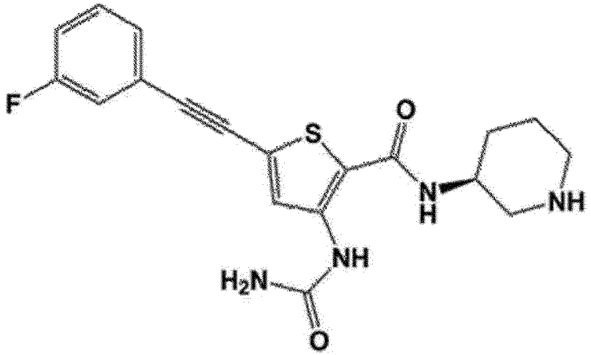

In one embodiment of the present invention, the anticancer drug may be a *bcl2* inhibitor.

In one embodiment of the present invention, the anticancer drug may be any one or more of cytarabine, venetoclax, and azacitidine.

Another aspect of the present invention provides a method for treating leukemia, comprising a step of administering the pharmaceutical composition to a leukemia patient.

Still another aspect of the present invention provides the use of a compound represented by the following Formula 1 for preventing or treating leukemia:

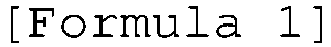

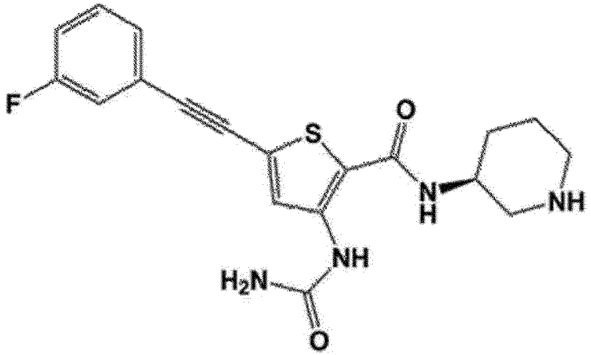

Yet another aspect of the present invention provides the use of a compound represented by the following Formula 1 for preparing a pharmaceutical composition for preventing or treating leukemia:

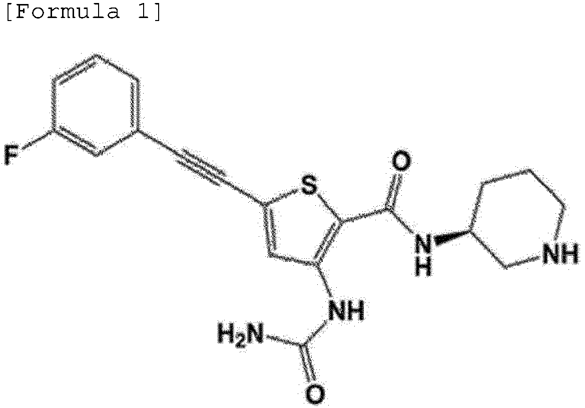

### Advantageous Effects

The composition for preventing, ameliorating or treating leukemia containing a 2,3,5-substituted thiophene compound and an anticancer drug according to one embodiment of the present invention has excellent inhibitory activity against cancer cells related to leukemia compared to treatment with the 2,3,5-substituted thiophene compound alone or the anticancer drug alone, and thus may be useful for the prevention, amelioration or treatment of leukemia.

### Brief Description of Drawings

FIG. 1 shows the results of confirming the optimal dosage upon administration of venetoclax alone.
FIG. 2 shows the results of confirming the optimal dosage upon administration of azacitidine alone.
Figure 3 shows the results of confirming the optimal dosage upon administration of cytarabine alone.
FIGS. 4 and 5 show the results of evaluating the effect of co-administration of the compound and venetoclax according to one embodiment of the present invention.
FIG. 6 shows the results of evaluating the effect of co-administration of the compound and azacitidine according to one embodiment of the present invention.
FIG. 7 shows the results of evaluating the effect of co-administration of the compound and cytarabine according to one embodiment of the present invention.

### Best Mode

One aspect of the present invention provides a pharmaceutical composition for preventing or treating leukemia, containing a compound represented by the following Formula 1 or a pharmaceutically acceptable salt thereof and an anticancer drug:

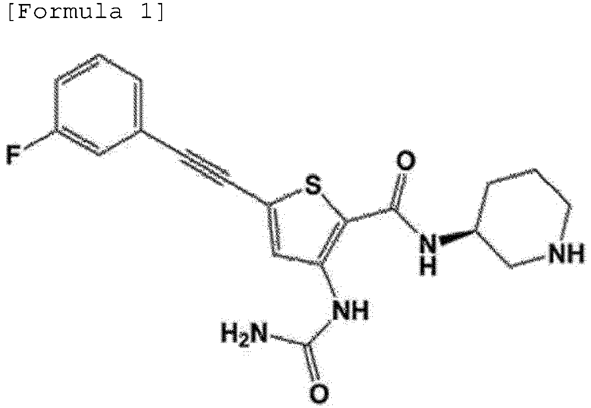

The compound represented by Formula 1, which is contained as an active ingredient in the pharmaceutical composition of the present invention, is (S)-5-((3-fluorophenyl)ethynyl)-N-(piperidin-3-yl)-3-ureido-thiophene-2-carboxamide.

In one embodiment of the present invention, the anticancer drug may be a *bcl2* inhibitor. The *bcl2* inhibitor refers to a substance that inhibits the expression and activity of B-cell lymphoma 2 (Bcl2) protein that regulates apoptosis by inducing pro-apoptosis and inhibiting anti-apoptosis. The *bcl2* inhibitor may be any one or more of cytarabine, venetoclax, and azacitidine.

In addition, cytarabine is a cytotoxic anticancer drug that inhibits DNA synthesis necessary for cell survival, and is known to be used alone or in combination to treat leukemia such as acute myeloid leukemia and various cancers.

Venetoclax (Venclexta^{®} or Venclyxto^{®}) is a substance that is used for the treatment of acute myeloid leukemia (AML), chronic lymphocytic leukemia (CLL), small lymphocytic lymphoma (SLL), etc.

Azacitidine (or 5-azacytidine) is known as a substance that penetrates RNA or DNA through the nucleic acid biosynthesis pathway in actively dividing cells, thereby inhibiting protein synthesis and exhibiting a cell-killing effect.

In one embodiment of the present invention, the dosage ratio of cytarabine to the compound represented by Formula 1 or pharmaceutically acceptable salt thereof may be 1:1 to 1:6,000,
the dosage ratio of venetoclax to the compound represented by Formula 1 or pharmaceutically acceptable salt thereof may be 1:0.0001 to 1: 100, and
the dosage ratio of azacitidine to the compound represented by Formula 1 or pharmaceutically acceptable salt thereof may be 1:0.02 to 1:14.

It was confirmed that the pharmaceutical composition for preventing or treating leukemia according to the present invention, which contains the compound represented by Formula 1 or pharmaceutically acceptable salt thereof in combination with an anticancer drug, exhibited an excellent synergistic effect, specifically, an excellent effect on treatment of leukemia.

The pharmaceutical composition of the present invention may be prepared in a unit dose form or prepared to be contained in a multi-dose container by formulating with a pharmaceutically acceptable carrier, according to a method that a person skilled in the art can easily perform.

Examples of the pharmaceutically acceptable carrier include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, which are commonly used in formulation. The pharmaceutical composition of the present invention may further contain a lubricant, a wetting agent, a sweetening agent, a flavoring agent, an emulsifying agent, a suspending agent, a preservative, and the like, in addition to the above-described components. Suitable pharmaceutically acceptable carriers and formulations are described in detail in Remington's Pharmaceutical Sciences (22nd ed., 2013).

In the present invention, the content of additives in the pharmaceutical composition is not particularly limited and may be appropriately adjusted within the content range used in conventional formulations.

The composition of the present invention may be formulated into injectable formulations such as aqueous solutions, suspensions, emulsions, etc., pills, capsules, granules, or tablets.

The pharmaceutical composition of the present invention may be for oral administration, and non-limiting examples of formulations for oral administration include tablets, troches, lozenges, aqueous suspensions, oily suspensions, prepared powders, granules, emulsions, hard capsules, soft capsules, syrups, or elixirs.

In addition, the pharmaceutical composition of the present invention may be for parenteral administration, and non-limiting examples of parenteral formulations include injection solutions, suppositories, powders for respiratory inhalation, aerosols for sprays, ointments, powders for application, oils, creams, etc.

A preferable dosage of the pharmaceutical composition of the present invention may vary depending on the patient's condition, body weight, age, sex, health status, dietary constitution specificity, the nature of the formulation, the severity of disease, administration time of the composition, administration method, administration period or interval, excretion rate, and drug form, and may be appropriately selected by those skilled in the art. For example, it may be in the range of about 0.1 to 10,000 mg/kg, but is not limited thereto and may be administered once or several times a day.

In the present invention, the pharmaceutical composition may be administered orally or parenterally (e.g., intravenously, subcutaneously, intraperitoneally, or topically) depending on a desired method.

The use of the compound represented by Formula 1 or pharmaceutically acceptable salt thereof in combination with the anticancer drug includes not only preparing them as a single formulation, but also preparing them as individual formulations which are administered together or at different times.

When the compound represented by Formula 1 or pharmaceutically acceptable salt thereof and the anticancer drug are prepared as a single formulation, the single formulation may be prepared by mixing the compound represented by Formula 1 or pharmaceutically acceptable salt thereof with the anticancer drug, or may be prepared so that the compound represented by Formula 1 or pharmaceutically acceptable salt thereof and the anticancer drug form different compartments.

When the compound represented by Formula 1 or pharmaceutically acceptable salt thereof and the anticancer drug are prepared as individual formulations, they may be formulated in different forms by considering the individual dosage, administration route, administration time, etc., depending on the characteristics of the compound represented by Formula 1 or pharmaceutically acceptable salt thereof and the anticancer drug.

In one embodiment of the present invention, the compound represented by Formula 1, a stereoisomer thereof, a pharmaceutically acceptable salt thereof, a hydrate thereof, or a solvate thereof may be administered parenterally or orally, preferably orally.

The pharmaceutically effective amount or effective dosage of the pharmaceutical composition of the present invention may vary depending on the formulation method, administration method, administration time, and/or administration route of the pharmaceutical composition, and a person skilled in the art can easily determine and prescribe an effective dosage for intended treatment.

The pharmaceutical composition of the present invention may be administered once or several times a day.

Another aspect of the present invention provides a method for treating leukemia, comprising a step of administering the pharmaceutical composition to a leukemia patient.

The pharmaceutical composition according to one embodiment of the present invention contains the compound represented by Formula 1 and an anticancer drug as active ingredients. Therefore, the method may further comprise a companion diagnosis step of selecting a group of patients on which the compound represented by Formula 1 and the anticancer drug show an effect, before administering the pharmaceutical composition of the present invention. This companion diagnosis step may be performed by a leukemia diagnostic method known in the art. As used herein, the term "companion diagnosis" refers to diagnosis for predicting a patient's response to a specific drug treatment.

Another aspect of the present invention provides the use of the compound represented by Formula 1 for preventing or treating leukemia or the use of the compound represented by Formula 1 for preparing a pharmaceutical composition for preventing or treating leukemia.

### Mode for Invention

Hereafter, the present invention will be described in more detail with reference to one or more examples. However, these examples are intended to illustrate one or more embodiments and the scope of the present invention is not limited to these examples.

### Experimental Example 1: Materials and Methods

The experimental materials and equipment used below are as follows:

**[Table 1]**

| **Materials and Equipment** | **Company** | **Cat #** |
|---|---|---|
| IMDM | Gibco | 12440-053 |
| RPMI1640 | Hyclone | SH30027.01 |
| CellTiterGlo viability test kit | Promega | G7572 |
| 100 mm cell culture dish | SPL | SPL 20101 |
| Lumitrac 96 well tissue culture plate | Corning | 3903 |
| Disposable tissue culture pipette | SPL | 9100591010 |
| Test tube 15 ml | SPL | 5005 |
| Test tube 50 ml | SPL | 50015 |
| WellMate dispenser | Thermo Fisher | Wellmate^{™} |
| Liquid handler | Perkin Elmer | Janus |
| Label reader | Perkin Elmer | Victor 3 |
| Venetoclax | Selleckchem | S8048 |
| 5-azacytidine | Sigma aldrich | A2385 |
| Cytarabine | Selleckchem | S1648 |
| Compound (PHI-101) of Formula 1 | Provided from PharosiBT | |

### Experimental Example 2: Confirmation of Optimal Dosages of Individual Anticancer Drugs

### Experimental Example 2-1. Confirmation of Dosage of Venetoclax

BDCM, HL-60, MolM13, MolM14, MV4-11, THP-1, and U937 were prepared as leukemia cell lines, and venetoclax was prepared as a drug to be administered.

The first and second tests were performed in triplicate on the above cell lines while reducing the concentration of venetoclax by half each time from 100 µM, and the third test was performed in triplicate on the BDCM cell line while reducing the concentration of venetoclax by half each time from 100 µM, and performed in triplicate on HL-60, MolM13, MolM14, and MV4-11 cell lines while reducing the concentration of venetoclax by half each time from 2.5 µM. The fourth test was performed in triplicate on HL-60, MolM13, MolM14, and MV4-11 cell lines while reducing the concentration by half each time from 2.5 µM.

Specifically, in the first and second tests, a 10-fold diluted solution was prepared by dissolving 50 µL of 10 mM venetoclax in 450 µL of solvent. 250 µL of the solution was serially diluted 1/2, an 8E-tube was filled with 250 µL of each dilution, and 80 µL of each cell line was dispensed into each well of a 96-well plate. Thereafter, 10 µL of each cell line was dispensed using JANUS and incubated for 72 hours under CO₂. Celltiter-Glo assay was performed and then IC₅₀ was calculated.

In the third test, for the BCDM cell line, a 10-fold diluted solution was prepared by dissolving 15 µL of 10 mM venetoclax in 135 µL of solvent. 100 µL of the solution was serially diluted 1/2, an 8E-tube was filled with 100 µL of each dilution, and the cell line was dispensed into a 96-well plate. Thereafter, 25 µL of the cell line was dispensed using JANUS and incubated for 72 hours under CO₂. Celltiter-Glo assay was performed and then IC₅₀ was calculated. Meanwhile, for the HL-60, MolM13, MolM14, and MV4-11 cell lines, a 10-fold diluted solution was prepared by dissolving 1 µL of 10 mM venetoclax in 399 µL of solvent. 200 µL of the solution was serially diluted 1/2, an 8E-tube was filled with 200 µL of each dilution, and each of the cell lines was dispensed into a 96-well plate. Thereafter, 60 µL of each cell line was dispensed using JANUS and incubated for 72 hours under CO₂. Celltiter-Glo assay was performed and then IC₅₀ was calculated.

The fourth test was performed on HL-60, MolM13, MolM14, and MV4-11 cell lines in the same manner as the third test.

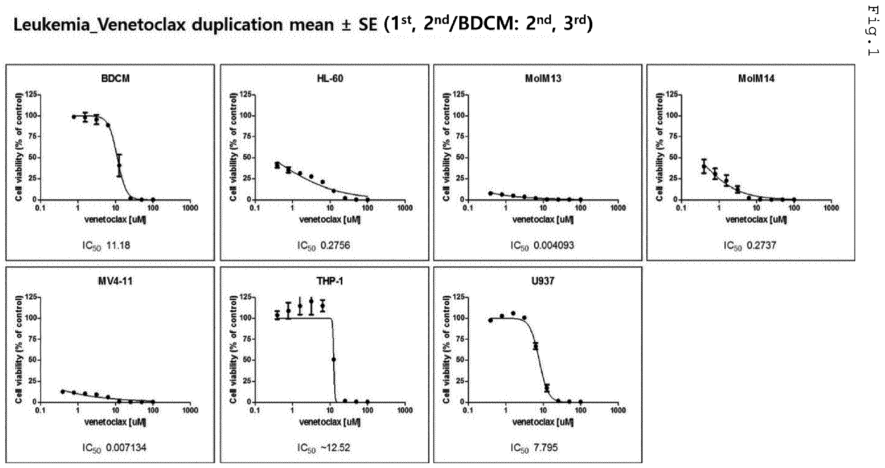

As a result of the above tests, as shown in FIG. 1 and Table 2 below, the IC₅₀ of venetoclax was confirmed. It was confirmed that venetoclax had an IC₅₀ of 1 µM or less in the HL-60, MolM13, MolM14, and MV4-11 leukemia cancer cell lines.

**[Table 2]**

| IC₅₀ | First test [µM] | Second test [µM] | Third test [µM] | Fourth test [µM] |
|---|---|---|---|---|
| BDCM | 12.25 | 9.857 | 12.75 | - |
| HL-60 | 0.3528 | 0.2105 | 0.283 | 0.2759 |
| MolM13 | 0.0018 | 0.0125 | 0.0086 | 0.0103 |
| MolM14 | 0.1723 | 0.4149 | 0.1467 | 0.2189 |
| MV4-11 | 0.0068 | 0.0087 | 0.0219 | 0.0141 |
| THP-1 | 12.53 | 12.5 | - | - |
| U937 | 7.312 | 8.319 | - | - |

### Experimental Example 2-2. Confirmation of Dosage of 5-Azacytidine

BDCM, HL-60, MolM13, MolM14, MV4-11, THP-1, and U937 were prepared as leukemia cell lines, and 5-azacytidine was prepared as a drug to be administered.

The first and second tests were performed in triplicate on the above cell lines while reducing the concentration of 5-azacytidine by half each time from 100 µM. Specifically, in the first and second tests, a 10-fold diluted solution was prepared by dissolving 15 µL of 20 mM 5-azacytidine in 285 µL of solvent. 250 µL of the solution was serially diluted 1/2, an 8E-tube was filled with 250 µL of each dilution, and 80 µL of each cell line (3,000 cells) was dispensed into each well of a 96-well plate. Thereafter, 10 µL of each cell line was dispensed using JANUS and incubated for 72 hours under CO₂. Celltiter-Glo assay was performed and then IC₅₀ was calculated.

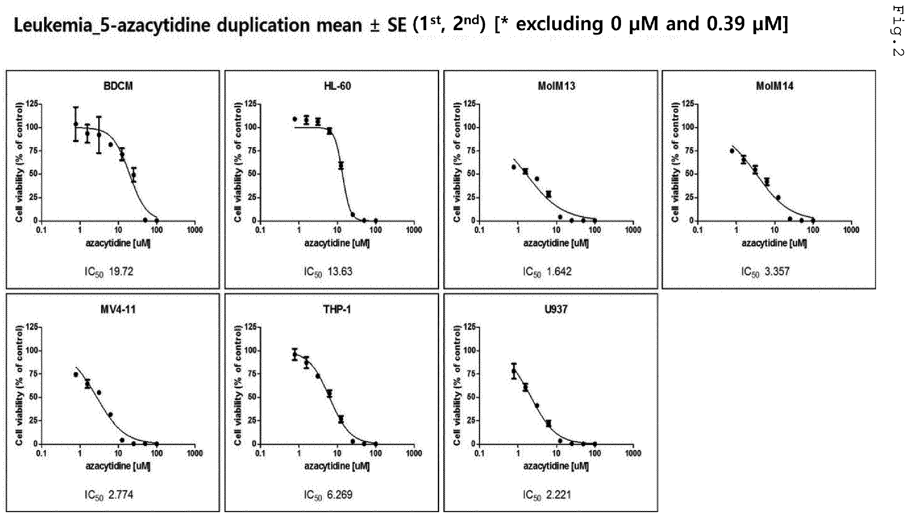

As a result, IC₅₀ values as shown in FIG. 2 and Table 3 below were obtained.

**[Table 3]**

| IC₅₀ | First test [µM] | Second test [uM] |
|---|---|---|
| BDCM | 14.81 | 23.61 |
| HL-60 | 14.7 | 13.16 |
| MolM13 | 1.706 | 1.41 |
| MolM14 | 3.879 | 2.824 |
| MV4-11 | 3.05 | 2.451 |
| THP-1 | 6.083 | 6.376 |
| U937 | 1.908 | 2.544 |

### Experimental Example 2-3. Confirmation of Dosage of Cytarabine

BDCM, HL-60, MolM13, MolM14, MV4-11, THP-1, and U937 were prepared as leukemia cell lines, and cytarabine was prepared as a drug to be administered.

The first and second tests were performed in triplicate on the above cell lines while reducing the concentration of cytarabine by half each time from 50,000 µM. Specifically, in the first and second tests, a 10-fold diluted solution was prepared by dissolving 10 µL of 50 mM cytarabine in 490 µL of solvent. 250 µL of the solution was serially diluted 1/2, an 8E-tube was filled with 250 µL of each dilution, and 80 µL of each cell line (3,000 cells) was dispensed into each well of a 96-well plate. Thereafter, 10 µL of each cell line was dispensed using JANUS and incubated for 72 hours under CO₂. Celltiter-Glo assay was performed and then IC₅₀ was calculated.

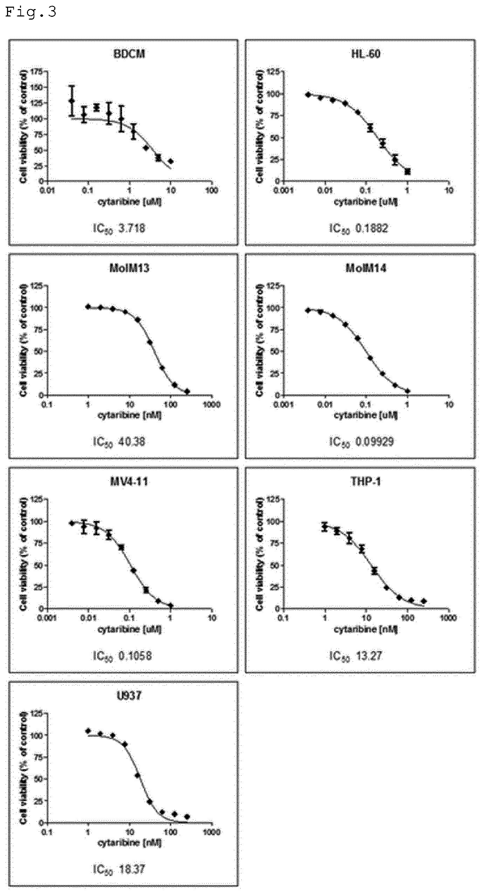

As a result, IC₅₀ values as shown in FIG. 3 and Table 4 below were obtained.

**[Table 4]**

| IC₅₀ | First test | Second test |
|---|---|---|
| BDCM | 2.826 µM | 4.482 µM |
| HL-60 | 0.2206 µM | 0.1626 µM |
| MolM13 | 38.99 nM | 41.8 nM |
| MolM14 | 0.099 µM | 0.0933 µM |
| MV4-11 | 0.1002 µM | 0.1078 µM |
| THP-1 | 11.22 nM | 15.27 nM |
| U937 | 18.34 M | 18.39 M |

### Experimental Example 3: Evaluation of Effect of Co-administration

### Experimental Example 3-1. Evaluation of Effect of Co-administration of Compound of Formula 1 and Venetoclax

BDCM, HL-60, MolM13, MolM14, MV4-11, THP-1, and U937 were prepared as leukemia cell lines, and PHI-101 and venetoclax were prepared as drugs to be administered.

Specifically, in the first and second tests, a 10-fold diluted solution was prepared by dissolving 1 µL of 1 mM venetoclax in 999 µL of solvent. 500 µL of the solution was serially diluted 1/2, an 8E-tube was filled with 500 µL of each dilution, and 90 µL of each cell line was dispensed into each well of a 96-well plate. Thereafter, 10 µL of each cell line was dispensed using JANUS and pretreated for 30 minutes under CO₂. Then, a PHI-101 solution was prepared as shown in Table 5 below, and 10 µL of the solution was added to each well of the plate. Next, the cells were incubated for 72 hours under CO₂, Celltiter-Glo assay was performed and then IC₅₀ was calculated.

**[Table 5]**

| | |
|---|---|
| BDCM | 1 mM PHI-101 2.3 µL + medium 997.7 µL |
| HL-60 | 10 mM PHI-101 2.2 µL + medium 997.8 µL |
| MolM13 | 10 µM PHI-101 2 µL + medium 1998 µL |
| MolM14 | 10 µM PHI-101 8 µL + medium 992 µL |
| MV4-11 | 10 µM PHI-101 2 µL + medium 1998 µL |
| THP-1 | 1 mM PHI-101 7.35 µL + medium 992.65 µL |
| U937 | 10 mM PHI-101 3.4 µL + medium 996.6 µL |

In the third and fourth tests, a 10-fold diluted solution was prepared by dissolving 5 µL of 50 mM PHI-101 in 495 µL of solvent. 250 µL of the solution was serially diluted 1/2, an 8E-tube was filled with 250 µL of each dilution, and 60 µL of each cell line was dispensed into each well of a 96-well plate. Thereafter, 10 µL of each cell line was dispensed using JANUS and pretreated for 30 minutes under CO₂. Then, a venetoclax solution was prepared as shown in Table 6 below, and 10 µL of the solution was added to each well of the plate. Next, the cells were incubated for 72 hours under CO₂, Celltiter-Glo assay was performed and then IC₅₀ was calculated.

**[Table 6]**

| | |
|---|---|
| BDCM | 10 mM Venetoclax 11 µL + medium 989 µL |
| HL-60 | 1 mM Venetoclax 2.75 µL + medium 997.25 µL |
| THP-1 | 10 mM Venetoclax 12.5 µL + medium 987.5 µL |
| U937 | 10 mM Venetoclax 7.8 µL + medium 992.2 µL |

The results obtained above were taken together and compared with those obtained when venetoclax or PHI-101 was administered alone.

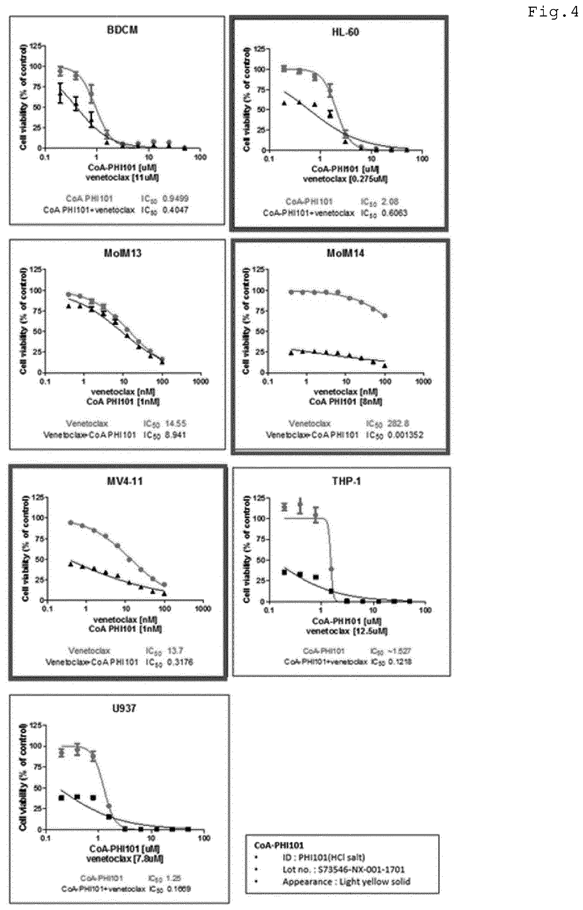

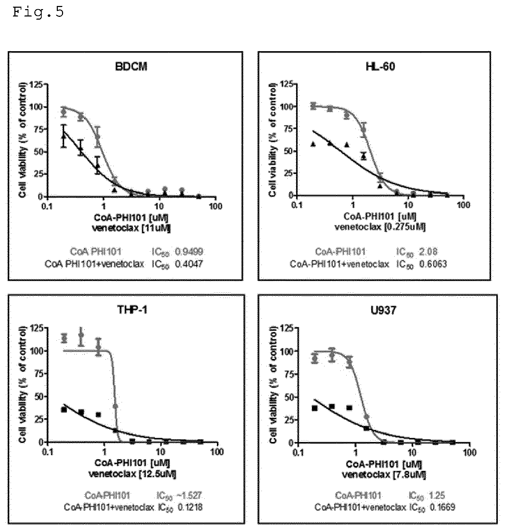

As a result, as shown in Table 7 below and FIGS. 4 and 5, it was confirmed that the effect obtained when venetoclax and PHI-101 were co-administered to each leukemia cancer cell line was 1.63- to 282,800-fold better than that obtained when venetoclax or PHI-101 was administered alone.

**[Table 7]**

| **Cell line** | **Venetoclax** | **PHI-101** | **Combination** | **Fold (PHI-101/Combination)** |
|---|---|---|---|---|
| BDCM | 11.0 µM | 0.950 µM | 0.405 µM | 27.2 |
| HL-60 | 0.275 µM | 2.08 µM | 0.606 µM | 0.453 |
| THP-1 | 12.5 µM | 1.527 µM | 0.122 µM | 102.5 |
| U937 | 7.8 µM | 1.25 µM | 0.167 µM | 46.7 |
| MolM 13 | 14.55 nM | 1.00 nM | 8.94 nM | 1.63 |
| MoIM 14 | 282.8 nM | 8.0 nM | 0.001 nM | 282,800 |
| MV4-11 | 13.7 nM | 1 nM | 0.318 nM | 43.1 |

### Experimental Example 3-2. Evaluation of Effect of Co-administration of Compound of Formula 1 and Azacitidine

BDCM, HL-60, MolM13, MolM14, MV4-11, THP-1, and U937 were prepared as leukemia cell lines, and 5-azacytidine and PHI-101 were prepared as drugs to be administered.

Specifically, in the first and second tests, for BDCM, HL-60, THP-1, and U937 cell lines, a 10-fold diluted solution was prepared by dissolving 5 µL of 50 mM PHI-101 in 495 µL of solvent. 250 µL of the solution was serially diluted 1/2, an 8E-tube was filled with 250 µL of each dilution, and 60 µL of each cell line (3,000 cells) was dispensed into each well of a 96-well plate. Thereafter, 10 µL of each cell line was dispensed using JANUS and pretreated for 30 minutes under CO₂. Then, a 5-azacytidine solution was prepared as shown in Table 8 below, and 10 µL of the solution was added to each well of the plate. Next, the cells were incubated for 72 hours under CO₂, Celltiter-Glo assay was performed and then IC₅₀ was calculated.

**[Table 8]**

| | |
|---|---|
| BDCM | 20 mM 5-azacytidine 9.5 µL + medium 990.5 µL |
| HL-60 | 20 mM 5-azacytidine 6.75 µL + medium 993.25 µL |
| THP-1 | 20 mM 5-azacytidine 3.15 µL + medium 986.85 µL |
| U937 | 20 mM 5-azacytidine 1.1 µL + medium 998.9 µL |

Meanwhile, for MolM13, MolM14, and MV4-11 cell lines, a 10-fold diluted solution was prepared by dissolving 1 µL of 1 mM PHI-101 in 999 µL of solvent. 250 µL of the solution was serially diluted 1/2, an 8E-tube was filled with 250 µL of each dilution, and 50 µL of each cell line (3,000 cells) was dispensed into each well of a 96-well plate. Thereafter, 10 µL of each cell line was dispensed using JANUS and pretreated for 30 minutes under CO₂. Then, a 5-azacytidine solution was prepared as shown in Table 9 below, and 10 µL of the solution was added to each well of the plate. Next, the cells were incubated for 72 hours under CO₂, Celltiter-Glo assay was performed and then IC₅₀ was calculated.

**[Table 9]**

| | |
|---|---|
| MolM13 | 20 mM 5-azacytidine 1.6 µL + medium 1,998.4 µL |
| MolM14 | 20 mM 5-azacytidine 1.7 µL + medium 998.3 µL |
| MV4-11 | 20 mM 5-azacytidine 1.4 µL + medium 998.6 µL |

The results obtained above were taken together and compared with those obtained when 5-azacytidine or PHI-101 was administered alone.

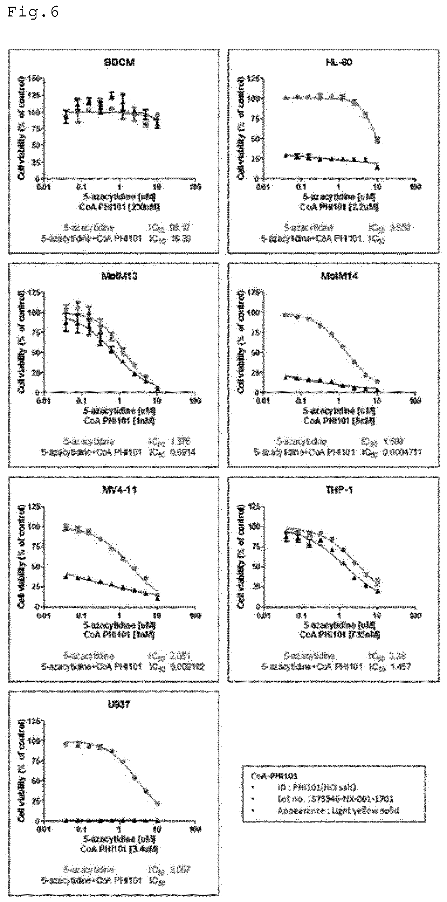

As a result, as shown in Table 10 below and FIG. 6, it was confirmed that the effect obtained when 5-azacytidine and PHI-101 were co-administered was 2- to 3,180-fold better than that obtained when 5-azacytidine or PHI-101 was administered alone. The treatment of the HL-60 and U937 leukemia cell lines with PHI-101 showed an inhibitory activity of 500 or less at the minimum concentration.

**[Table 10]**

| **Cell line** | **Azacitidine** | **PHI-101** | **Combination** | **Fold (PHI-101/Combination)** |
|---|---|---|---|---|
| BDCM | 98.17 µM | 0.230 µM | 16.39 µM | 6.0 |
| HL-60 | 9.66 µM | 2.2 µM | - | - |
| MolM 13 | 1.38 µM | 0.001 µM | 0.69 µM | 2.0 |
| MoIM 14 | 1.59 µM | 0.008 µM | 0.0005 µM | 3180.0 |
| MV4-11 | 2.05 µM | 0.001 µM | 0.009 µM | 227.8 |
| THP-1 | 3.38 µM | 0.735 µM | 1.457 µM | 2.3 |
| U937 | 3.06 µM | 3.4 µM | - | - |

### Experimental Example 3-3. Evaluation of Effect of Co-administration of Compound of Formula 1 and Cytarabine

BDCM, HL-60, MolM13, MolM14, MV4-11, THP-1, and U937 were prepared as leukemia cell lines, and cytarabine and PHI-101 were prepared as drugs to be administered.

Specifically, in the first and second tests, for BDCM, HL-60, THP-1, and U937 cell lines, a 10-fold diluted solution was prepared by dissolving 5 µL of 50 mM PHI-101 in 495 µL of solvent. 250 µL of the solution was serially diluted 1/2, an 8E-tube was filled with 250 µL of each dilution, and 60 µL of each cell line (3,000 cells) was dispensed into each well of a 96-well plate. Thereafter, 10 µL of each cell line was dispensed using JANUS and pretreated for 30 minutes under CO₂. Then, a cytarabine solution was prepared as shown in Table 11 below, and 10 µL of the solution was added to each well of the plate. Next, the cells were incubated for 72 hours under CO₂, Celltiter-Glo assay was performed and then IC₅₀ was calculated.

**[Table 11]**

| | |
|---|---|
| BDCM | 10 mM Cytarabine 1.23 µL + medium 998.77 µL |
| HL-60 | 100 µM Cytarabine 6.3 µL + medium 993.7 µL |
| THP-1 | 10 µM Cytarabine 4.3 µL + medium 995.7 µL |
| U937 | 10 µM Cytarabine 6 µL + medium 994 µL |

Meanwhile, for MolM13, MolM14, and MV4-11 cell lines, a 10-fold diluted solution was prepared by dissolving 1 µL of 1 mM CoA PHI-101 in 999 µL of solvent. 250 µL of the solution was serially diluted 1/2, an 8E-tube was filled with 250 µL of each dilution, and 50 µL of each cell line (3,000 cells) was dispensed into each well of a 96-well plate. Thereafter, 10 µL of each cell line was dispensed using JANUS and pretreated for 30 minutes under CO₂. Then, a cytarabine solution was prepared as shown in Table 12 below, and 10 µL of the solution was added to each well of the plate. Next, the cells were incubated for 72 hours under CO₂, Celltiter-Glo assay was performed and then IC₅₀ was calculated.

**[Table 12]**

| | |
|---|---|
| MolM13 | 100 µM Cytarabine 4 µL + medium 996 µL |
| MolM14 | 100 µM Cytarabine 10 µL + medium 990 µL |
| MV4-11 | 100 µM Cytarabine 10 µL + medium 990 µL |

The results obtained above were taken together and compared with those obtained when cytarabine or PHI-101 was administered alone.

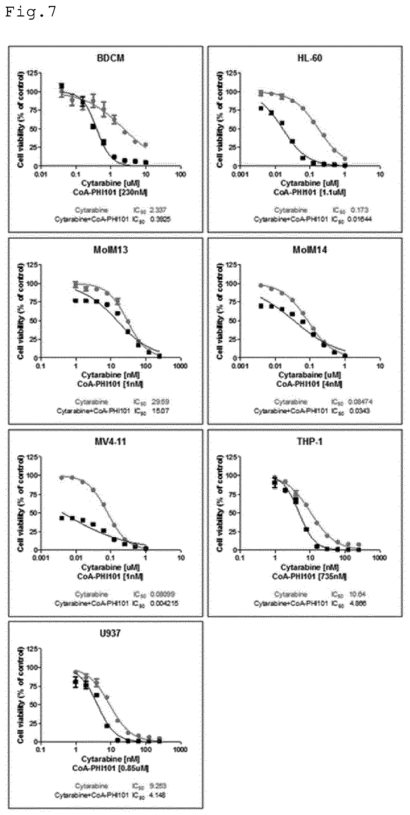

As a result, as shown in Table 13 below and FIG. 7, it was confirmed that the effect obtained when cytarabine and PHI-101 were co-administered was 2- to 19.2-fold better than that obtained when cytarabine or PHI-101 was administered alone.

**[Table 13]**

| **Cell line** | **Cytarabine** | **PHI-101** | **Combination** | **Fold (Cytarabine/Combination)** |
|---|---|---|---|---|
| BDCM | 2.34 µM | 0.23 µM | 0.39 µM | 6.0 |
| HL-60 | 0.17 µM | 1.1 µM | 0.016 µM | 10.6 |
| MolM 13 | 29.59 nM | 1.0 nM | 15.07 nM | 2.0 |
| MoIM 14 | 84.74 nM | 4.0 nM | 34.3 nM | 2.5 |
| MV4-11 | 80.99 nM | 1.0 nM | 4.215 nM | 19.2 |
| THP-1 | 10.64 nM | 735.0 nM | 4.87 nM | 2.2 |
| U937 | 9.25 nM | 850.0 uM | 4.15 nM | 2.2 |

So far, the present invention has been described with reference to the embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A pharmaceutical composition for preventing or treating leukemia, containing a compound represented by the following Formula 1 or a pharmaceutically acceptable salt thereof and an anticancer drug:

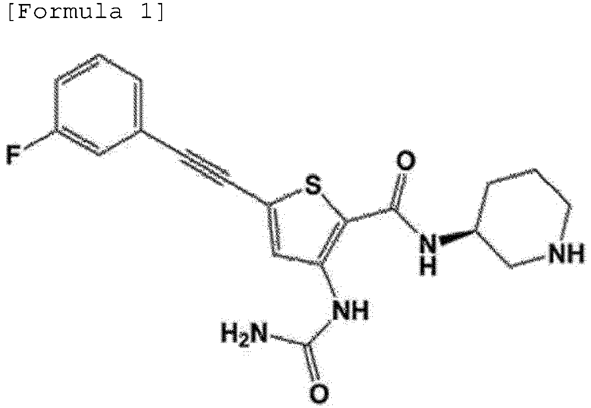

2. The pharmaceutical composition for preventing or treating leukemia according to claim 1, wherein the anticancer drug is a *bcl2* inhibitor

3. The pharmaceutical composition for preventing or treating leukemia according to claim 1, wherein the anticancer drug is any one or more of cytarabine, venetoclax, and azacitidine.

4. A method for treating leukemia, comprising a step of administering the pharmaceutical composition of claim 1 to a leukemia patient.

5. Use of a compound represented by the following Formula 1 for preventing or treating leukemia:

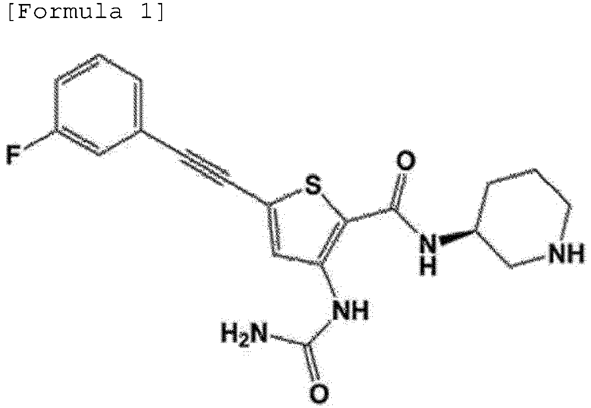

6. Use of a compound represented by the following Formula 1 for preparing a pharmaceutical composition for preventing or treating leukemia:
